# EUROPEAN PATENT SPECIFICATION

(11) **EP 0 862 399 B1**
(45) Date of publication and mention of the grant of the patent: **22.02.2006**
(21) Application number: 97937218.2
(22) Date of filing: 13.08.1997
(51) Int. Cl.: A61F 11/12, H04R 1/10, A61F 11/14

(54) **A DEVICE HAVING ROTATABLE LEGS**
VORRICHTUNG MIT DREHBAREN SCHENKELN
DISPOSITIF A BRANCHES ROTATIVES

(30) Priority: 15.08.1996 US 698367; 19.12.1996 US 769930
(43) Date of publication of application: 09.09.1998
(73) Proprietor: Cabot Safety Intermediate Corporation, Southbridge, MA 01550 (US)
(72) Inventor: FALCO, Robert, N., Indianapolis, IN 46268 (US); DOTY, Mark, Brownsburg, IN 46112 (US); MYERS, Brian, Indianapolis, IN 46236 (US)
(74) Representative: Leckey, David Herbert
(86) International application number: PCT/US1997/014216
(87) International publication number: WO 1998/006363

(56) References cited:
- WO-A-96/15541
- FR-A- 2 078 583
- US-A- 1 066 511
- US-A- 1 483 315
- US-A- 2 420 245
- US-A- 3 970 082
- US-A- 4 461 290
- US-A- 4 671 265
- US-A- 4 689 822
- US-A- 5 188 123
- PATENT ABSTRACTS OF JAPAN vol. 1995, no. 03, 28 April 1995 (1995-04-28) & JP 06 351090 A (SONY CORP), 22 December 1994 (1994-12-22)
- PATENT ABSTRACTS OF JAPAN vol. 009, no. 075 (E-306), 4 April 1985 (1985-04-04) & JP 59 210790 A (MATSUSHITA DENKI SANGYO KK), 29 November 1984 (1984-11-29)
- PATENT ABSTRACTS OF JAPAN vol. 008, no. 139 (E-253), 28 June 1984 (1984-06-28) & JP 59 047896 A (MATSUSHITA DENKI SANGYO KK), 17 March 1984 (1984-03-17)

## Description

The invention relates generally to hearing protection devices and in particular to a hearing protection device including rotatable legs for adjusting the position of hearing protectors.

### Prior Art:

JP-A-06351090 and JP-A-59210790 describe foldable headphone units.

Hearing protectors fall generally into three categories, including protectors that cap the entrance to the ear canal; protectors which enter the ear canal and seal the ear canal prior to the bend in the ear canal (usually referred to simply as semi-insert devices); and protectors that enter the ear canal and take the bend in the ear canal (sometimes referred to as banded earplugs). US, 4,671,265 describes an earplug device including a headband with earplugs attached to the ends thereof. US 2 420 245 discloses an ear muff having swivel joints permitting links holding the muffs to move inwardly and outwardly. Semi-insert hearing protectors generally protect similarly to earplugs, but usually to a lesser level. Semi-insert hearing protectors are also referred to as semi-aural hearing protectors. Semi-insert hearing protectors which enter the ear canal to a greater degree offer better protection but are somewhat less comfortable than those which simply cap the ear. Products which cap the ear have some of the attributes of both earplugs and earmuffs. Typically, they are used for intermittent noise exposures where lighter weight and improved low frequency attenuation are desirable. As used herein, "hearing protectors" refers generally to hearing protectors falling into one of the three categories described above.

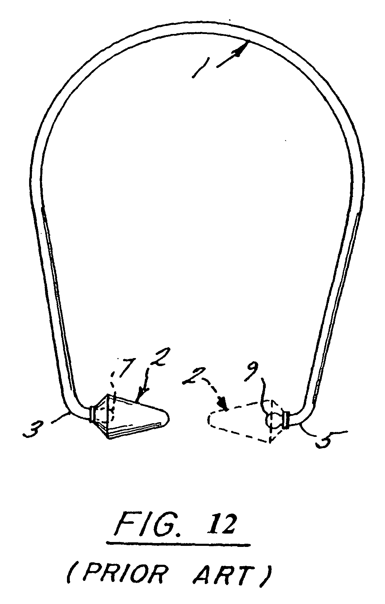

FIGURE 12 is a front view of a conventional hearing protection device disclosed in U.S. Patent 4,461,290. The hearing protection device includes a U-shaped headband 1 having affixed to each of the opposed end portions 3 and 5 an inwardly directed ear obturating pod 2. A pair of balls 7 and 9 engage a socket formed in the pods 2 and attach the pods 2 to the headband 1. While well-suited for its intended purposes, the conventional headband 1 may have certain drawbacks and deficiencies. For example, the shape of the headband is such that when the pods 2 are mounted in the wearer's ears, portions of the headband 1 are close to or touching the wearer's head. This can be irritating and uncomfortable to the wearer. In addition, there is no mechanism for adjusting the headband to allow for varying head sizes. A wearer with a large head requires a large distance between the headband ends 3 and 5. Unfortunately, as the distance between the headband ends 3 and 5 increases, so does the tension in the headband 1. Accordingly, wearers with large heads may experience discomfort due to this high tension in the headband 1. In order to accommodate varying head sizes, the headband 1 must be manufactured in multiple sizes which increases manufacturing costs. Accordingly, there is a perceived need in the art for a hearing protection device that can comfortably fit a wide variety of head sizes.

### Summary of the Invention:

The above-discussed and other drawbacks and deficiencies of the prior art are overcome or alleviated in accordance with the present invention by the hearing protection device as claimed in claim 1. In a preferred embodiment at least, the hearing protection device includes a frame having a band and a pair of legs. The pair of legs are rotatably coupled to the ends of the band. Each leg has a first leg end rotatably coupled to a respective band end and a second leg end for receiving a hearing protector. In a preferred embodiment, each second end has a mating means (preferably an opening) for receiving a stem portion of the hearing protector.

The hearing protector in accordance with this invention provides several important features and advantages relative to the prior art. For example, the rotatable legs allow the band to accommodate virtually any head size and provide the wearer with both comfort and a variety of wearing positions.

The above-discussed features and advantages of the present invention will be appreciated and understood by those skilled in the art from the following description and drawings.

### Brief Description of the Drawings:

Referring now to the drawings wherein like elements are numbered alike in the several FIGURES:
FIGURE 1 is a front view of a frame of a hearing protection device in a first embodiment of the invention;
FIGURE 2 is a top view of the frame of FIGURE 1;
FIGURE 3 is a bottom view of the frame of FIGURE 1;
FIGURE 4 is a side view of the frame of FIGURE 1;
FIGURE 5 is a view taken along line 5-5 in FIGURE 1;
FIGURE 6 is a cross-sectional view of one end of a band shown in FIGURE 1;
FIGURE 7 is a cross-sectional view of a leg shown in FIGURE 1;
FIGURE 8 is a front view of the frame of FIGURE 1 fitted with a first type hearing protector;
FIGURE 9 is a front view of the frame of FIGURE 1 fitted with a second type of hearing protector;
FIGURE 10 is a front view of the frame of FIGURE 1 fitted with a third type of hearing protector;
FIGURES 11A-11B are a cross-sectional views of a hearing protectors for use with the hearing protection device of FIGURE 1;
FIGURE 12 is a front view of a conventional hearing protection device;
FIGURES 13A-C are perspective views depicting the hearing protective device of this invention in three different positions on a wearer's head;
FIGURES 14A-14C are front views of the frame fitted with a variety of hearing protectors;
FIGURE 15 is a cross-sectional view of a frequency attenuating hearing protector for use with the present invention;
FIGURE 16 is an exploded view of an alternative system for mounting a hearing protector to the frame;
FIGURE 17 is a partial cross-sectional view of a hearing protector mounted to the frame using the system of FIGURE 16.

### Detailed Description of the Invention:

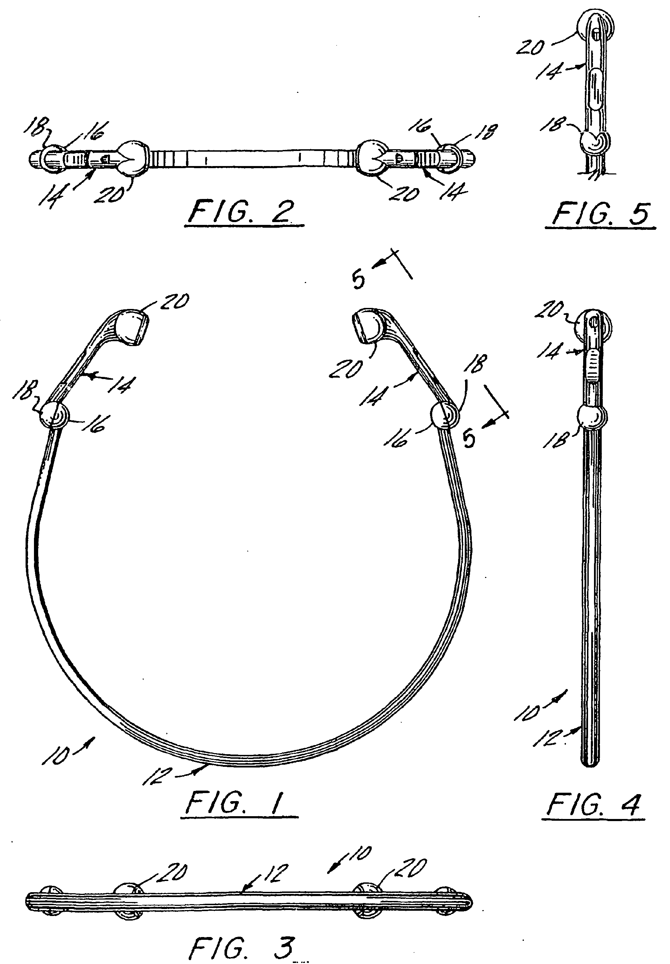

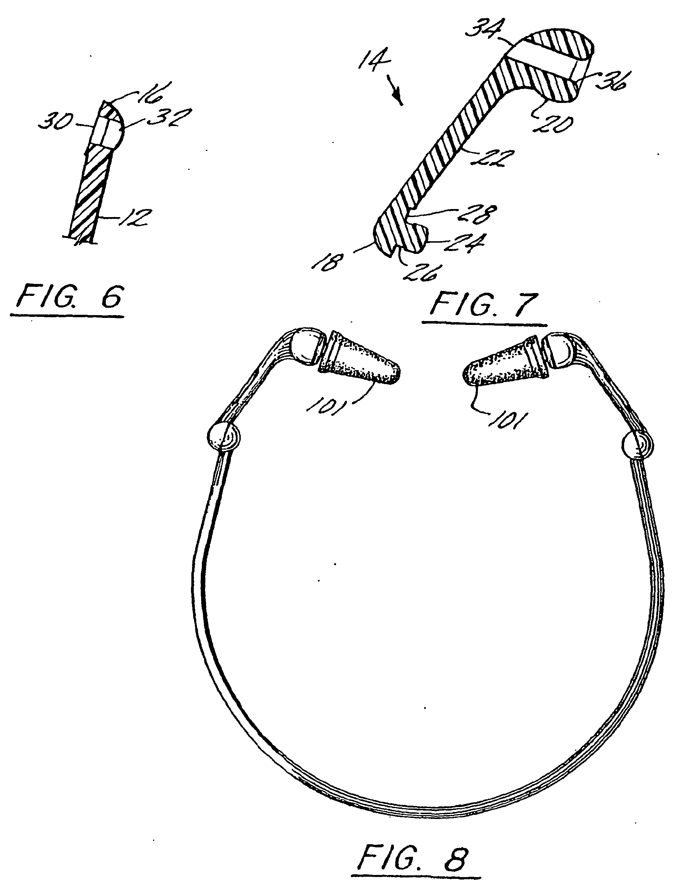

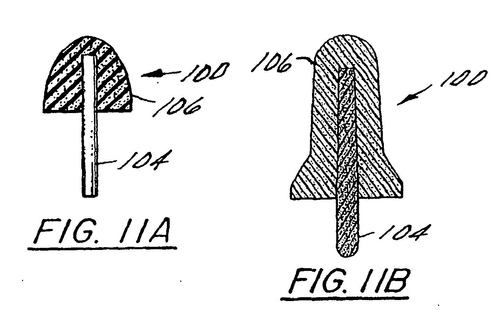

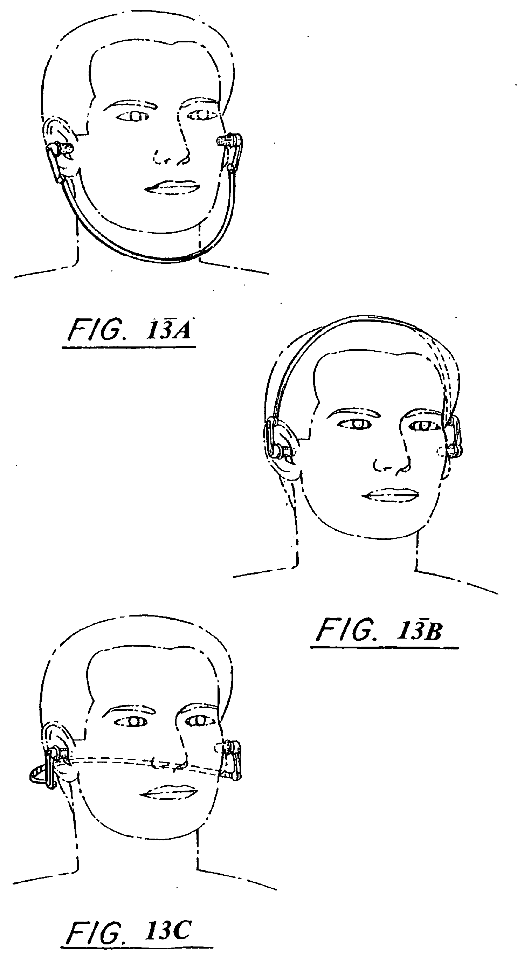

FIGURE 1 is a front view of the frame of the hearing protection device in a first embodiment of the invention. The frame is shown generally at 10. The frame 10 includes a band 12 and a pair of legs shown generally at 14. The band 12 is generally U-shaped and is made from a resilient, flexible material such as plastic. The band 12 has two leg supports 16, each of which forms one half of a swivel or rotating joint between the band 12 and the leg 14. The leg support 16 is integral with the band 12 and in an exemplary embodiment is a spherical segment. The leg 14 includes a first leg end 18 which is integral with leg 14 and, in an exemplary embodiment, is a spherical segment. The flat faces of the first leg end 18 and the leg support 16 are placed opposite each other to form a swivel joint. The details of the swivel joint are described below with reference to FIGURES 6 and 7. The swivel joint allows the first leg end 18 to rotate relative to the leg support 16 thereby allowing the leg 14 to rotate relative to the band 12. The band 12 lies substantially in a plane and as the legs 14 are rotated, a plurality of angles are defined between the leg 14 and the plane. It is understood that the band 12 and leg supports 16 need not lie in the same plane as shown in FIGURE 4. Indeed, as shown in FIGURES 13 A, B and C (and discussed hereafter) the leg supports 16 may lie in a plane different than the band 12 and each leg 14 will rotate through a plurality of angles between the leg 14 and the plane of the leg supports 16. The leg 14 includes a second leg end 20 for receiving a hearing protector 100 (shown in FIGURES 11A and 11B). In an exemplary embodiment, the second leg end 20 is a spherical segment. It will be appreciated that second leg end 20 may comprise any other desired functional shape such as ovular, triangular, etc. The outer dimension of the second leg end 20 is preferably equal to the average concha bowl opening so that the second leg end 20 cannot be inserted into the ear canal but is preferably insertable (and rests in) the concha bowl (so that the earplug attached to end 20 is permitted to freely articulate in the ear canal). FIGURES 2-5 are various views of the frame of the hearing protection device of FIGURE 1 and are self-explanatory.

FIGURE 6 is a cross-sectional view of the leg support 16 and FIGURE 7 is a cross-sectional view of the leg 14. The leg 14 includes first leg end 18, second leg end 20 and leg body 22. The first leg end 18 has a planar surface 28 and a post 24 substantially perpendicular to the planar surface 28. The post 24 has a neck region 26 where the diameter of the post 24 is smaller than the diameter of the end of the post 24 away from the planar surface 28. The leg support 16 includes a hole 32 formed therethrough for receiving the post 24. The neck 26 snap-lockedly engages a ridge 30 formed in the hole 32. The ridge 30 has an inner diameter that is smaller than the inner diameter of the hole 32. That is, the ridge 30 extends away from the inner wall of the hole 32. The interference fit between the neck 26 and the ridge 30 holds the leg 14 to the band 12 while allowing the post 24 to rotate within the hole 32. Because the band 12 and the leg 14 are made from a resilient material, such as plastic, the interference fit between the neck 26 and the ridge 30 can be defeated by pulling the post 24 out of the hole 32 if necessary.

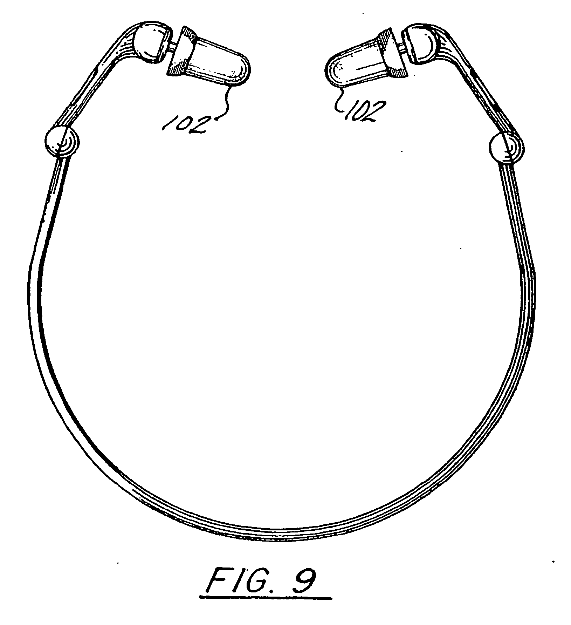

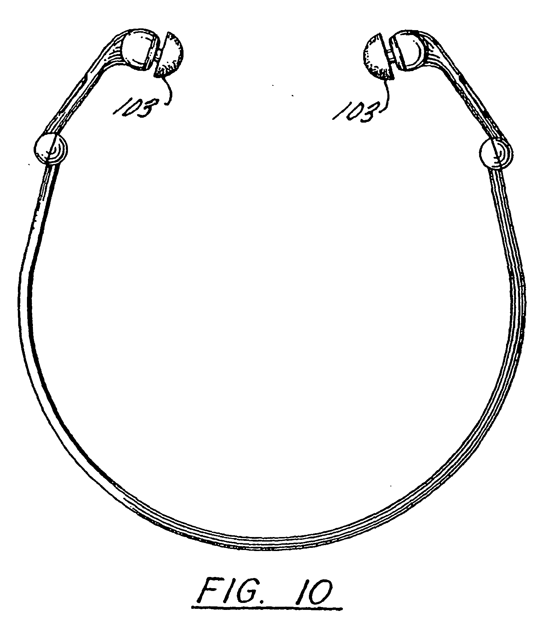

The planar surface 28 of the first leg end 18 is at an oblique angle relative to the major axis of the leg body 22. This causes the leg 14 to be angled towards the interior of the U-shaped band 12 as shown in FIGURE 1. This results in the second leg ends 20, which hold the hearing protectors as shown in FIGURES 8-10, being close to the wearer's head and the band 12 being away from the wearer's head. Because the band 12 is positioned away from the wearer's head, it is less likely that the band 12 will contact and irritate the wearer. Thus, the comfort of the hearing protection device is improved over the prior art.

In addition, as the legs 14 are rotated, the distance between the second leg ends 20 varies. Therefore, the tension on the band 12 can remain approximately the same for varying head sizes. In the device shown in FIGURE 12, the only way to adjust the distance between the hearing protectors 2 is to flex the band 1. This results in increasing tension as the wearer's head size increases. The frame 10 shown in FIGURE 1 allows the distance between the hearing protectors to be adjusted without flexing the band 12. Thus, wearers with different head sizes can achieve approximately the same tension in the band 12. In this way, the frame 10 can comfortably accommodate head sizes ranging from, for example, the 5th percentile of women to the 95th percentile of men. The legs 14 may be rotated while the hearing protection device is being worn to allow modification of the fit without removing the hearing protection device. In addition, the hearing protection device can be worn with the band 12 under the chin (as shown in FIGURE 13A), over the top of the head (as shown in FIGURE 13B), or behind the back of the head based on the wearer's preference (as shown in FIGURE 13C).

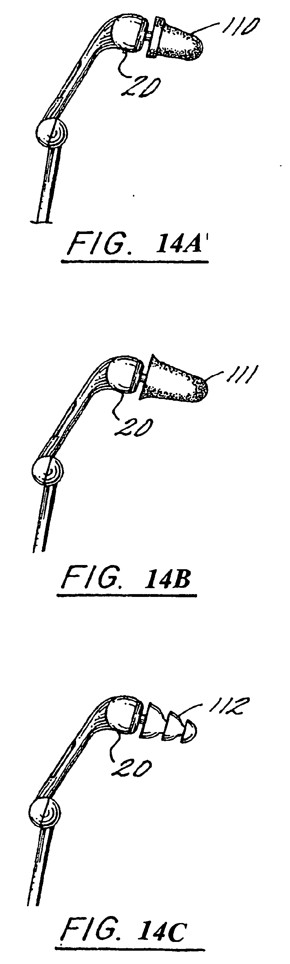

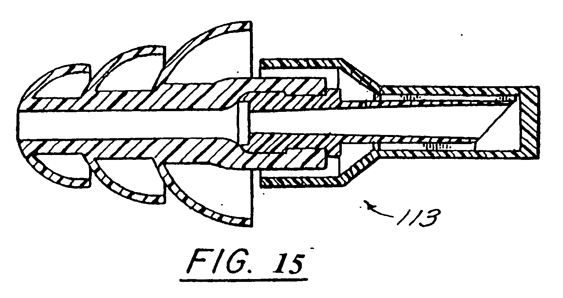

The second leg end 20 includes a hole 34 formed therein for receiving various hearing protectors 101-103 as shown in FIGURES 8-10. As mentioned above, the hearing protectors may be protectors that cap the entrance to the ear canal; protectors which enter the ear canal and seal the ear canal prior to the bend in the ear canal; or protectors that enter the ear canal and take the bend in the ear canal. FIGURES 14A-14C illustrate additional hearing protectors 110-112 that may be mounted to the second leg end 20. FIGURE 15 is a cross-sectional view of a hearing protector 113 that may be mounted to the second leg end 20. The hearing protector 113 attenuates sound over a wide frequency range uniformly. Additional details of the hearing protector in FIGURE 15 are provided in U.S. Patent 5,113,967.

The hearing protection device of the present invention provides a comfortable fit regardless of the size or shape of the hearing protectors mounted in the second leg ends 20.

As shown in FIGURES 11A and 11B, a hearing protector 100 comprises a foam portion 106 coupled to a stem 104. The hearing protector in FIGURE 11A is a conventional hearing protector in which the foam portion 106 is glued to the stem 104. The hearing protector shown in FIGURE 11B is an improved design in which the stem 104 is made from a porous material and the foam 106 interpenetrates the pores in the stem 104 to form a mechanical bond. This hearing protector shown in FIGURE 11B is described in additional detail below.

In an exemplary embodiment, the foam portion 106 is a dynamically stiff foam. A suitable dynamically stiff foam is described in commonly assigned U.S. Patent 5,420,381. Alternatively, the foam portion 106 may comprise any other conventional earplug foam material such as the foam disclosed in U.S. Reissue Patent 29,487.

The stem 104 is placed in the hole 34. The use of hole 34 and stem mounted hearing protector 100 allows the wearer to change hearing protectors easily. Hole 34 includes a tapered area 36 (shown in FIGURE 7) that eases placement of the hearing protector 100 in hole 34. The stem 104 is preferably flexible so that it flexes as the wearer moves their head. A non-pliable stem may cause discomfort as the wearer moves their head. By using a flexible stem 104, an extra degree of freedom is introduced into the hearing protection device. The foam portion 106 moves along with the wearer's head and provides additional comfort.

To provide a tight fit in the hole 34, the stem 104 should be made from a compressible, resilient material and have a diameter slightly larger than the diameter of hole 34. When the stem 104 is positioned in hole 34, the stem 104 will press against the wall of hole 34 and provide a friction fit. The hole 34 need not be formed completely through the second leg end 20 for the stem 104 to be securely mounted to the second leg end 20. However, if the hole 34 is formed all the way through the second leg end 20, the stem 104 can be pushed out of the second leg end 20 to aid removal of the hearing protector 100 if necessary.

As previously mentioned with reference to FIGURE 11B, in an exemplary embodiment, the stem 104 is made from a porous material, such as that disclosed in the U.S. Patent 5,799,658 filed contemporaneously herewith entitled "Hearing Protective Device Comprising a Foam and a Porous Component, and Method of Manufacturing Thereof". The porous material is both flexible and compressible. The porous material is preferably a polymer or combination of polymers in the form of small particles of one or more size ranges bonded in such a way as to leave voids or pores within the material. The pores may be of varying sizes. Preferably, the pore volume is in the range from about ten percent to about ninety percent of the total volume, with the most preferred pore volume being in the range from about thirty to about sixty percent of the total volume. A particularly preferred porous component is Swab-002 made from INTERFLO®, available from Interflo Technologies, Brooklyn, NY. The foam 106 interpenetrates the pores in the porous stem 104 to mechanically bond the foam portion 106 to the stem 104.

It will be appreciated that an ear protective device may be mounted to the ends of legs 14 in any suitable manner in addition to the hole 34 with mating stem 104 approach shown in the preferred embodiments. For example, a foam or other earplug device could be attached using a ball and socket configuration as in prior art FIGURE 12, or any other approach may be used whereby the earplug is attached to leg 14.

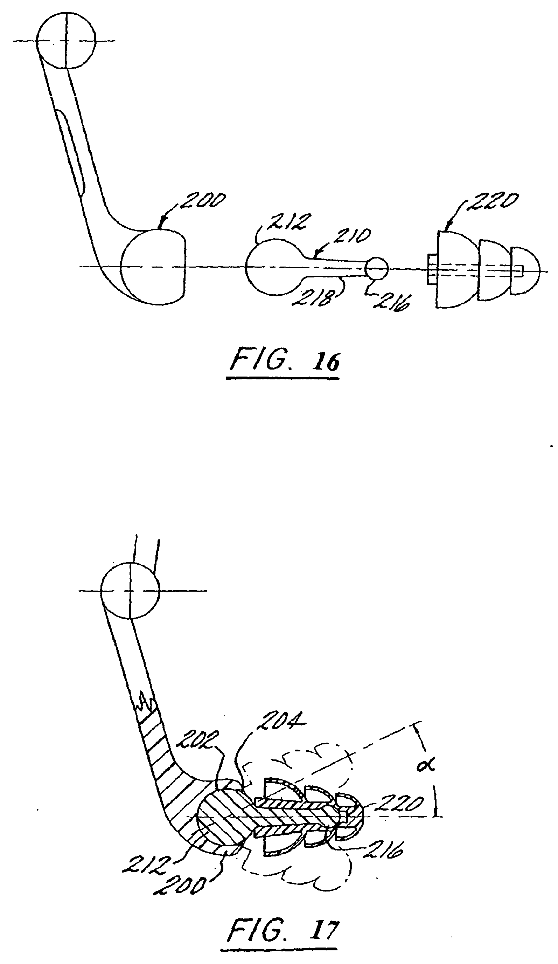

FIGURE 16 is an exploded view of an alternative system for mounting a hearing protector to the frame. A shaft 210 includes a first ball 212 and a second ball 216 joined by a shaft neck 218. The first ball 212 engages a socket 202 (shown in FIGURE 17) formed in the frame end 200. The socket 202 may be formed in the second leg end 20 shown in FIGURE 1. The second ball 216 engages a hearing protector 220. The hearing protector 220 shown in FIGURE 16 is a multiple flange earplug similar to the earplug described in U.S. Patent 4,867,149. It is understood, however, that any hearing protector may be configured for use with shaft 210.

FIGURE 17 is a partial cross sectional view of the hearing protector 220 mounted to the frame end 200 through shaft 210. The first ball 212 is placed within socket 202. The socket 202 includes a lip 204 that extends into the socket 202 to reduce the diameter of the opening of the socket 202. The outer diameter of the first ball 212 is slightly smaller than the inner diameter of the socket 202. The frame end 200 is made from a resilient material (e.g., plastic) and the lip 204 flexes to allow the first ball 212 to be placed within the socket 202. The lip 204 then returns to its original position once the first ball 212 is placed in the socket 202. The interference fit between the lip 204 and the first ball 212 retains the shaft 210 in the frame end 200. As previously mentioned, either the second leg end 20 or the reverse curve end 20' may include the socket 202 and lip 204. The hearing protector 220 is then placed on the shaft 210 and retained on the shaft by the interference fit between the second ball 216 and the hearing protector 220.

The mounting system of FIGURES 16 and 17 allows the hearing protector 220 to rotate way from a center line of the socket 202 by an angle α. FIGURE 17 illustrates the hearing protector 220 in three different positions. In an exemplary embodiment, a equals 27°. The use of the ball and socket mounting system shown in FIGURES 16 and 17 introduces additional degrees of freedom in the hearing protection device. This allows the wearer to finely adjust the position of the hearing protectors for a more comfortable fit.

The present invention provides a hearing protection device that comfortably fits head sizes ranging from the 5th percentile of women to the 95th percentile of men. This is accomplished through legs having a first end rotatably coupled to a band and a second end for receiving hearing protectors. As the legs are rotated, the distance between the hearing protectors varies. This allows wearers with differing head sizes to achieve the substantially the same band tension. To increase comfort, the hearing protector may be mounted on a flexible stem which allows the hearing protector to move relative to the leg. Alternatively, a ball and socket mounting system may be used to allow the wearer to finely adjust the position of the hearing protectors relative to the frame.

While preferred embodiments have been shown and described, various modifications and substitutions may be made thereto without departing from the scope of the invention. Accordingly, it is to be understood that the present invention has been described by way of illustration and not limitation.

## Claims

1. A hearing protection device comprising:
a band (12) having a pair of ends (16);
a pair of legs (14), each of said legs (14) having a first end (18) rotatably coupled to one of said band ends (16) and a second end (20) for receiving a hearing protector (100); and
a hearing protector (100) coupled to the second end (20) of each leg (14), said legs (14) being rotatable with respect to said band (12) while said hearing protection device is worn and wherein each of said legs (14) further comprises a leg body (22) connecting said first end (18) and said second end (20), **characterised by** said first ends (18) further comprising a planar surface (28) at an oblique angle relative to said leg body (22).

2. The hearing protection device of claim 1 wherein the hearing protector (100) includes a stem (104) and said second end (20) has a second hole (34) formed therein for receiving the stem (104).

3. The hearing protection device of claim 1 or 2, wherein each of said second ends (20) includes a socket (202) formed therein.

4. The hearing protection device of claim 1 further comprising a shaft (210) having a ball (212) on a first shaft end and said hearing protector (100) coupled to a second shaft end (216), wherein said second end (20) includes a socket (202) for receiving said ball.

5. The hearing protection device of any preceding claim wherein said band (12) lies substantially in a plane and an angle exists between said each of said legs (14) and said plane, said angle varying as said leg (14) is rotated.

6. The hearing protection device of any preceding claim wherein said band ends (16) each includes a first hole (32) formed therein and each of said first ends (18) includes a post (24) for engaging said first hole (32) to rotatably couple said first end (18) to said band end (16).

7. The hearing protection device of claim 6 wherein said post (24) extends away from and perpendicular to said planar surface (28).

8. The hearing protection device of any of claims 6 or 7 wherein said post (24) comprises a neck (26) having a diameter less than the diameter at a tip of said post (24), and said first hole (32) includes a ridge (30) having a diameter smaller than the diameter of said first hole (32), wherein said ridge (30) engages said neck (26) when said post (24) is placed within said first hole (32).

9. The hearing protection device of claim 2 wherein said second hole (34) is formed completely through said second end (20).

## Patentansprüche

1. Gehörschutzvorrichtung, mit:
einem Band (12), das ein Paar Enden (16) aufweist,
einem Paar von Schenkeln (14), wobei jeder der Schenkel (14) ein erstes Ende (18), das drehbar mit einem der Bandenden (16) gekoppelt ist, und ein zweites Ende (20) zum Aufnehmen eines Gehörschützers (100) aufweist, und
einem Gehörschützer (100), der mit dem zweiten Ende (20) jedes Schenkels (14) gekoppelt ist, wobei die Schenkel (14) drehbar in bezug auf das Band (12) sind, während die Gehörschutzvorrichtung getragen wird, und
wobei jeder der Schenkel (14) des weiteren einen Schenkelkörper (22) umfasst, der das erste Ende (18) und das zweite Ende (20) verbindet, **dadurch gekennzeichnet, dass**
die ersten Enden (18) des weiteren eine ebene Oberfläche (28) in einem schiefen Winkel relativ zu dem Schenkelkörper (22) umfassen.

2. Gehörschutzvorrichtung nach Anspruch 1, bei der der Gehörschützer (100) einen Stiel (104) umfasst, und das zweite Ende (20) ein darin ausgebildetes zweites Loch (34) aufweist, um den Stiel (104) aufzunehmen.

3. Gehörschutzvorrichtung nach Anspruch 1 oder 2, bei der jedes der zweiten Enden (20) eine darin ausgebildete Fassung bzw. Gelenkpfanne (202) umfasst.

4. Gehörschutzvorrichtung nach Anspruch 1, die des weiteren einen Schaft (210) umfasst, der eine Kugel (212) an einem ersten Schaftende und den mit einem zweiten Schaftende (216) gekoppelten Gehörschützer (100) aufweist, wobei das zweite Ende (20) eine Fassung bzw. Gelenkpfanne (202) zum Aufnehmen der Kugel umfasst.

5. Gehörschutzvorrichtung nach einem der voranstehenden Ansprüche, bei der das Band (12) im wesentlichen in einer Ebene liegt, und ein Winkel zwischen jedem der Schenkel (14) und der Ebene existiert, wobei der Winkel variiert, wenn der Schenkel (14) gedreht wird.

6. Gehörschutzvorrichtung nach einem der voranstehenden Ansprüche, bei der die Bandenden (16) jeweils ein darin ausgebildetes erstes Loch (32) umfassen, und jedes der ersten Enden (18) einen Stab (24) zum Eingreifen mit dem ersten Loch (32) umfasst, um das erste Ende (18) mit dem Bandende (16) drehbar zu koppeln.

7. Gehörschutzvorrichtung nach Anspruch 6, bei der sich der Stab (24) weg von und senkrecht zu der ebenen Oberfläche (28) erstreckt.

8. Gehörschutzvorrichtung nach Anspruch 6 oder 7, bei der der Stab (24) eine Verjüngung bzw. Einschnürung (26) umfasst, die einen Durchmesser aufweist, der kleiner als der Durchmesser einer Spitze des Stabs (24) ist, und bei der das erste Loch (32) eine Kante (30) umfasst, die einen Durchmesser aufweist, der kleiner als der Durchmesser des ersten Lochs (32) ist, wobei die Kante (30) in die Verjüngung bzw. Einschnürung (26) eingreift, wenn der Stab (24) innerhalb des ersten Lochs (32) angeordnet ist.

9. Gehörschutzvorrichtung nach Anspruch 2, bei der das zweite Loch (34) vollständig durch das zweite Ende (20) hindurch ausgebildet ist.

## Revendications

1. Dispositif de protection de l'ouïe comprenant :
un arceau (12) possédant une paire d'extrémités (16);
une paire de branches (14), chacune desdites branches (14) possédant une première extrémité (18) reliée de manière pivotante à l'une desdites extrémités (16) de l'arceau et une seconde extrémité (20) pour recevoir un dispositif (100) de protection de l'ouïe ; et
un dispositif (100) de protection de l'ouïe relié à la seconde extrémité (20) de chaque branche (14), lesdites branches (14) pouvant pivoter par rapport audit arceau (12) lorsque ledit dispositif de protection de l'ouïe est porté, et dans lequel chacune desdites branches (14) comprend également un corps de branche (22) qui relie ladite première extrémité (18) et ladite seconde extrémité (20), **caractérisé par le fait que** lesdites premières extrémités (18) possèdent en outre une surface plane (28) formant un angle oblique par rapport audit corps de branche (22).

2. Dispositif de protection de l'ouïe selon la revendication 1, dans lequel le dispositif (100) de protection de l'ouïe comprend une tige (104) et ladite seconde extrémité (20) possède un second orifice (34) formé intérieurement pour recevoir la tige (104).

3. Dispositif de protection de l'ouïe selon l'une quelconque des revendications 1 ou 2, dans lequel chacune desdites secondes extrémités (20) comprend un logement (202) formé en creux.

4. Dispositif de protection de l'ouïe selon la revendication 1, comprenant en outre une tige (210) qui possède une rotule (212) à une première extrémité de la tige et ledit dispositif (100) de protection de l'ouïe relié à une seconde extrémité (216) de la tige, dans lequel ladite seconde extrémité (20) comprend un logement (202) pour recevoir ladite rotule.

5. Dispositif de protection de l'ouïe selon l'une quelconque des revendications précédentes, dans lequel ledit arceau (12) s'étend sensiblement dans un plan et un angle existe entre chacune desdites branches (14) et ledit plan, ledit angle variant lorsque ladite branche (14) est pivotée.

6. Dispositif de protection de l'ouïe selon l'une quelconque des revendications précédentes, dans lequel lesdites extrémités (16) de l'arceau comprennent chacune un premier orifice (32) formé intérieurement, et chacune desdites premières extrémités (18) comprend un pilier (24) pour venir en prise avec le premier orifice (32) pour relier de manière pivotante ladite première extrémité (18) à ladite extrémité (16) de l'arceau.

7. Dispositif de protection de l'ouïe selon la revendication 6, dans lequel ledit pilier (24) s'étend perpendiculairement à ladite surface plane (28).

8. Dispositif de protection de l'ouïe selon l'une quelconque des revendications 6 ou 7, dans lequel ledit pilier (24) comprend une collerette (26) dont le diamètre est inférieur au diamètre extrémal dudit pilier (24), et ledit premier orifice (32) comprend une nervure (30) dont le diamètre est inférieur au diamètre dudit premier orifice (32), dans lequel ladite nervure (30) vient en prise avec ladite collerette (26) lorsque ledit pilier (24) est placé à l'intérieur dudit premier orifice (32).

9. Dispositif de protection de l'ouïe selon la revendication 2, dans lequel ledit second orifice (34) traverse entièrement ladite seconde extrémité (20).
